# DEMANDE DE BREVET EUROPEEN

(11) **EP 3 598 786 A1**
(43) Date de publication de la demande: **22.01.2020**
(21) Numéro de dépôt: 19186509.6
(22) Date de dépôt: 16.07.2019
(51) Int. Cl.: H04W 12/08, H04W 4/48, B64D 11/00, H04L 29/06, H04W 12/00

(54) **CONTINUITE D'ACCES AUX DONNEES AVIONIQUES HORS DU COCKPIT D'UN AERONEF**

(30) Priorité: 20.07.2018 FR 1800789
(71) Demandeur: Thales, 92400 Courbevoie (FR)
(72) Inventeur: CASTET, Laurent, 31100 TOULOUSE (FR); ERBA, Renaud, 31100 TOULOUSE (FR); SANCHEZ, Frédéric, 31100 TOULOUSE (FR)
(74) Mandataire: Lucas, Laurent Jacques

(57) **Abrégé**

L'invention concerne des systèmes et des procédés pour la communication de données de type avionique à un dispositif de type non-avionique situé en dehors du cockpit d'un aéronef. Un procédé peut comprendre les étapes consistant à recevoir une requête visant à recevoir des données avioniques depuis des systèmes avioniques de la part d'un dispositif client non-avionique; déterminer la distance entre le dispositif non-avionique et le cockpit de l'aéronef; et ajuster l'envoi de données en réponse à ladite requête en fonction de la distance déterminée. Des développements de l'invention décrivent l'utilisation d'un point d'accès sans-fil associé à une passerelle sécurisée déterminant les droits d'accès aux données avioniques par le dispositif non-avionique, divers ajustement d'envoi et/ou d'affichage en fonction notamment de la distance, la gestion de seuils, différentes techniques de positionnement en intérieur, des communications unilatérales ou bilatérales, diverses notifications, l'utilisation de clients légers connectés (e.g. oreillette, montre, lunettes, etc).

## Description

### Domaine de l'invention

L'invention concerne le domaine aéronautique en général et plus particulièrement des procédés et des systèmes pour l'accès continu aux données avioniques hors du cockpit, la gestion de données avioniques et la distribution d'affichages ainsi que la gestion de paramètres de vol émises par un dispositif non-avionique.

### Etat de la Technique

L'accès aux données avioniques (données soumises à un haut niveau d'intégrité et de sécurité et qui sont manipulées par les nombreuses fonctions ou systèmes permettant d'assurer la conduite du vol) n'est généralement possible que depuis le cockpit où sont rassemblées toutes les interfaces qui permettent d'interagir avec ces fonctions et systèmes.

Si le pilote sort du cockpit pour une raison quelconque (e.g. période de repos lors d'un vol long-courrier par exemple, toilettes, etc), il n'a plus accès à ces données, créant ainsi une rupture dans la réalisation de sa mission. Il existe donc des délimitations spatiales qui conditionnent l'accès aux données désirées.

A bord d'un aéronef, il existe plusieurs endroits dans lesquels des interfaces de consultation et/ou d'édition de données existent. Ces endroits peuvent être dispersés spatialement et peuvent générer de nombreux déplacements (fatigue, perte de temps, risque d'erreur, dérangement des passagers, etc) ou se révéler gênant dans la disposition de la cabine, voire trop accessibles aux passagers.

Les pilotes ou copilotes ne sont en effet pas les seuls à avoir besoin d'accéder à des données avioniques. Le personnel navigant doit aussi parfois interagir avec les fonctions et sous-systèmes avioniques, ce qui peut engendrer beaucoup de déplacements dans l'avion. Ces interactions peuvent provoquer de la fatigue, du stress, et peut en outre déranger les passagers dans la cabine. Autrement dit, la mobilité dans l'aéronef peut être contradictoire avec l'efficacité des prises de décision, du fait de limitation d'actions inscrites dans l'espace.

Ces problèmes techniques n'ont actuellement pas de solutions techniques. Certains documents de brevet décrivent l'utilisation d'écrans de passagers (acronyme IFE pour « In Flight Entertainment »). Ces approches comportent des limitations (e.g. disponibilité des écrans, confidentialité et sécurité, etc).

Le document de brevet US9284045 décrit des techniques pour simplifier le fonctionnement et la maintenance d'un aéronef en configurant une unité d'avionique pour communiquer sans fil et recevoir des données liées à l'avionique de manière transparente. Dans des modes de réalisation, un aéronef est équipé d'un dispositif avionique certifié réglementaire configuré pour être installé dans l'aéronef. Le système comprend un lecteur de carte mémoire et une carte mémoire associée. La carte mémoire comprend un émetteur-récepteur sans fil pour faciliter la communication entre l'unité avionique et un dispositif mobile (par exemple, un ordinateur, une tablette ou un téléphone intelligent employant une application appropriée, un dispositif avionique portable, etc.). Cette approche comporte des limitations.

Il existe un besoin pour des procédés et des systèmes avancés pour l'accès continu en temps réel aux données avioniques hors du cockpit, notamment en termes de consultation et de modification de données avioniques.

### Résumé de l'invention

L'invention concerne des systèmes et des procédés pour la communication de données de type avionique à un dispositif de type non-avionique situé en dehors du cockpit d'un aéronef. Un procédé peut comprendre les étapes consistant à recevoir une requête visant à recevoir des données avioniques depuis des systèmes avioniques de la part d'un dispositif client non-avionique, déterminer la distance entre le dispositif non-avionique et le cockpit de l'aéronef; et ajuster l'envoi de données en réponse à ladite requête en fonction de la distance déterminée. Des développements de l'invention décrivent l'utilisation d'un point d'accès sans-fil associé à une passerelle sécurisée déterminant les droits d'accès aux données avioniques par le dispositif non-avionique, divers ajustements d'envoi et/ou d'affichage en fonction notamment de la distance, la gestion de seuils, différentes techniques de positionnement en intérieur, des communications unilatérales ou bilatérales, diverses notifications, l'utilisation de clients légers connectés (e.g. oreillette, montre, lunettes, etc).

Avantageusement, les modes de réalisation de l'invention seront utilisés dans des avions de lignes commerciaux ou militaires par les différents personnels en charge, notamment les pilotes, les PNC (Personnel Navigant Commercial) et les agents de maintenance.

Avantageusement, en particulier lors de des vols de longue durée, les modes de réalisation de l'invention permettent une connectivité continue des utilisateurs avec les systèmes avioniques.

Avantageusement, les modes de réalisation selon l'invention permettent une grande mobilité et liberté de mouvement, du fait de l'utilisation de clients sécurisés légers, ergonomiques et peu intrusifs.

Avantageusement les modes de réalisation de l'invention améliorent la sécurité et la sûreté du vol en permettant au pilote de garder le contact avec la mission opérationnelle à chaque instant, et donc même en dehors du cockpit.

Avantageusement, les modes de réalisation de l'invention peuvent contribuer à réduire la charge de travail est donc les risques d'erreurs humaines.

Avantageusement, les modes de régulation de l'invention peuvent améliorer la sécurité du vol par l'apport d'une continuité de mission (dans le temps et dans l'espace), tout en limitant les activités à moindre valeur ajoutée et en réduisant les risques d'erreurs humaines.

Avantageusement, les modes de réalisation de l'invention permettent de synchroniser les différents personnels dispersés dans l'avion sur une même information, fiable et cohérente. Par ailleurs, de nouvelles possibilités d'interaction entre les personnels navigants deviennent possibles.

Avantageusement, les modes de réalisation selon l'invention permettent une présentation optimisés des différentes données selon le dispositif porté. En effet, les interfaces utilisateur peuvent être adaptées aux dimensions et aux résolutions d'écrans accessibles considérés dans leur ensemble (e.g. coopération entre écrans i.e. distribution spatiale de l'affichage).

Avantageusement, les modes de réalisation de l'invention permettent l'utilisation des données du monde ouvert (e.g. croisement de données, corrélation, inférence, application de règles logiques, etc.), en plus des données de type avionique. Les données combinées peuvent ainsi être enrichies et améliorer la sûreté et ou la sécurité aéronautique (information consolidée de haut niveau à forte valeur ajoutée)

Avantageusement, un mode de réalisation de l'invention permet d"accéder à des informations fusionnées et synthétiques, fondées sur des données qui normalement ne peuvent être consultées qu'à travers des médias spécifiques, non co- localisés et sans possibilité de les superposer simplement pour en tirer une information consolidée et pertinente.

En particulier l'invention permet de fusionner des données issues du monde avionique (calculateurs et fonctions embarqués dans l'avion) avec des données issues du monde ouvert (« Open World », pouvant provenir du sol ou d'autres avions). Les données accessibles recouvrent donc aussi bien le monde avionique que le monde ouvert.

De plus la présentation des données peut être améliorée afin de fournir l'information la plus ergonomique, la plus synthétique et la plus pertinente possible. L'invention permet l'interaction déportée entre les utilisateurs et les fonctions du monde avionique, ainsi qu'entre les utilisateurs eux-mêmes.

Avantageusement, les modes de réalisation permettent de réduire le stress de l'équipage grâce aux fonctions de notification sur les clients légers, évitant des déplacements inutiles dans l'avion (jusque-là imposés pour aller vérifier à la source des informations e.g. entre le cockpit et la cabine). Les modes de réalisation de l'invention peuvent permettre de nouvelles interactions entre membres d'équipage.

Avantageusement, certains modes de réalisation de l'invention permettent de diminuer les activités à faible valeur ajoutée, en fournissant des informations enrichies (le traitement de données est automatisé i.e. effectué en partie par les systèmes avioniques, lesquels se chargent notamment de traiter et d'assimiler les informations à l'état brut ou de bas niveau).

Avantageusement, les modes de réalisation de l'invention permettent de diminuer les risques d'erreurs humaines.

Les modes de réalisation de l'invention permettent généralement de réaliser des gains d'exploitation (focalisation des utilisateurs sur la réalisation d'activités à forte valeur ajoutée).

### Description des figures

D'autres caractéristiques et avantages de l'invention apparaîtront à l'aide de la description qui suit et des figures des dessins annexés dans lesquels:
La figure 1 illustre certains aspects de l'architecture générale des modes de réalisation de l'invention ;
La figure 2 illustre un mode de réalisation de l'invention, dans lequel la distance dans l'espace entre le cockpit et un client léger est manipulée pour l'accès aux données avioniques;
La figure 3 illustre des exemples d'interface utilisateur sur une montre connectée selon un mode de réalisation de l'invention ;
La figure 4 illustre l'envoi d'une commande de vol ou d'un paramètre de vol depuis un plusieurs clients légers à l'avionique via la passerelle sécurisée et le point d'accès.

### Description détaillée de l'invention

Certains termes et environnements techniques sont définis ci-après.

Un aéronef est un moyen de transport capable d'évoluer au sein de l'atmosphère terrestre. Par exemple, un aéronef peut être un avion ou un hélicoptère (ou bien encore un drone).

Un « système avionique » (ou « système de type avionique ») est un système présentant des caractéristiques *techniques* spécifiques en comparaison d'un système « non-avionique » (ou « système de type non-avionique » ou « monde ouvert »), ces caractéristiques techniques étant certifiées *administrativement* par une autorité de surveillance de la sécurité (en l'occurrence le régulateur aéronautique).

L'aéronef comprend une cabine de pilotage et des soutes avioniques. Au sein de ces dernières se trouvent des équipements avioniques de pilotage et de navigation installés par l'avionneur (certifiés par le régulateur aéronautique au sein d'un certificat de type dit « TC »), des équipements avioniques installés par l'équipementier aéronautique (certifié par le régulateur aéronautique au sein d'un certificat de type supplémentaire dit « STC ») et des équipements optionnels non-avioniques dont l'usage est approuvé par le régulateur aéronautique au sein d'une approbation opérationnelle dite « Ops Approval »).

Concernant les caractéristiques techniques distinctives d'un système avionique, un système - de manière générale, i.e. avionique ou non-avionique - peut présenter ou être associé avec un taux de défaillance prédéfini (parmi une plage de taux de défaillance prédéfinie), un taux de défaillance comprenant ou déterminant un taux d'erreur d'exécution prédéfini.

Dans un mode de réalisation, le taux de défaillance d'un système de type avionique est inférieur au taux de défaillance d'un système de type non-avionique. Dans un mode de réalisation, le taux de défaillance d'un système avionique est significativement ou substantiellement inférieur à celui d'un système non-avionique.

Un système avionique désigne un système fiabilisé (ou à fiabilité garantie). C'est un système dont la défaillance a des conséquences excédant des limites acceptées ou acceptables, donc redoutées. Une défaillance peut se caractériser par la perte de la fonction considérée, ou par la production de données erronées, avec ou sans détection d'une erreur. Selon le niveau de criticité des conséquences redoutées, la probabilité d'occurrence doit être maintenue inférieure à un seuil d'acceptabilité. Ainsi, plus la conséquence est critique, plus la probabilité d'occurrence acceptable est faible. Par exemple, en aéronautique, un événement catastrophique (décès multiples) devra avoir une probabilité d'occurrence inférieure à 10^-9 par heure de vol, alors qu'un incident majeur (réduction des marges de sécurité et des capacités opérationnelles, inconfort ou blessures légères) devra avoir une probabilité d'occurrence inférieure à 10^-5 par heure de vol. Pour assurer ces objectifs, l'architecture du système avionique (fiabilisé) ainsi que la conception de chaque composant garantissent cette probabilité d'occurrence par des garanties de taux de panne de chaque équipement (pannes physiques) et des niveaux de vérification (couverture fonctionnelle et structurelle de test) des logiciels.

Ces exigences imposent un effort important de conception et de vérification, et imposent une limitation dans la complexité des traitements mis en oeuvre.

A l'inverse, la défaillance d'un système non-fiabilisé, ou à la fiabilité non garantie (système non-avionique) a des conséquences jugées tolérables, non critiques, ou même sans impact opérationnel significatif. Les exigences sur l'architecture, les composants physiques ou les traitements logiciels sont donc moindres, et autorisent des traitements plus complexes, et un effort de développement et de vérification réduits par rapport à un système fiabilisé.

De manière générale, un système avionique est associé avec un taux de panne physique inférieur et une vérification logique supérieure à ceux d'un système de type non avionique.

Des modes de réalisation sont décrits ci-après.

Concernant notamment *l'architecture générale de gestion et d'accès aux données hors cockpit,* il est décrit un procédé pour la communication de données de type avionique à un dispositif de type non-avionique situé en dehors du cockpit d'un aéronef, le procédé comprenant les étapes consistant à : recevoir une requête visant à recevoir des données de type avionique depuis un ou plusieurs systèmes avioniques situés à un point de référence dans l'aéronef de la part d'un dispositif client non-avionique; déterminer un paramètre de liaison entre le dispositif client non-avionique et le point de référence; ajuster l'envoi de données en réponse à ladite requête en fonction du paramètre de liaison déterminé.

Dans un mode de réalisation, il est décrit un procédé pour la communication de données de type avionique à un dispositif de type non-avionique situé en dehors du cockpit d'un aéronef, le procédé comprenant les étapes consistant à : recevoir une requête visant à recevoir des données de type avionique depuis un ou plusieurs systèmes avioniques de la part d'un dispositif client non-avionique; déterminer un paramètre de liaison, comme la distance entre le dispositif non-avionique et le cockpit d'un aéronef (e.g. de l'aéronef, ou du cockpit d'un avion escorteur), ajuster l'envoi de données en réponse à ladite requête en fonction de la distance déterminée.

Dans un mode de réalisation, le paramètre de liaison comprend un ou plusieurs paramètres d'espace et/ou de temps caractérisant la relation entre le dispositif client non-avionique et un ou plusieurs systèmes avioniques, le paramètre de liaison comprenant notamment la distance physique entre le dispositif client non-avionique et le point de référence dans l'aéronef, et/ou la qualité de service associée aux communications sans-fil entre le dispositif client non-avionique et le point de référence dans l'aéronef, et/ou d'un droit d'accès prédéfini associé à un dispositif avionique et/ou au dispositif client non-avionique.

Selon les modes de réalisation, le paramètre de liaison peut varier. Le paramètre de liaison qualifie la relation entre le dispositif non-avionique et un autre point de référence spatial dans l'aéronef. Le paramètre peut être de nature espace et/ou temps. Le dispositif non-avionique peut être associé ou relatif à l'utilisateur. Le paramètre de liaison peut donc également comprendre par exemple le grade de l'utilisateur et/ou la séniorité de l'utilisateur qui utilise le dispositif (gestion des droits d'accès). Le paramètre de liaison peut comprendre la localisation du dispositif par rapport à un point de référence, lequel peut être le cockpit mais également d'autres parties de l'aéronef comme une porte de secours, un local de repos, un siège passager ou PNC, etc. Le paramètre de liaison peut comprendre des indicateurs de qualité de service (QoS), comme la stabilité et/ou de débit des communications à bord de l'aéronef (e.g. qualité de connexion, décalage en temps ou *lag*, etc). Dans un mode de réalisation, le paramètre de liaison comprend un ou plusieurs critères temporels (e.g. certaines réponses doivent être réalisées selon des intervalles de temps prédéfinis, ni trop tôt, ni trop tard, ce qui peut indirectement témoigner d'une réponse émanant d'une personne humaine et/ou constituer un secret associé au mode de communication. Dans un mode de réalisation, le paramètre de liaison est un paramètre synthétique, i.e. qui combine ou agrège plusieurs des paramètres énoncés précédemment.

Dans un mode de réalisation, l'aéronef peut être un drone télé-piloté et des conditions portant sur le paramètre de liaison peuvent être critiques (par exemple la distance déterminée entre le poste de commande et le drone à un instant donné peut conditionner l'envoi et/ou la réception de données). Les communications le cas échéant sont réalisées par HF, VHF ou SatCom.

Dans un mode de réalisation, le point de référence est le cockpit de l'aéronef. Dans un mode de réalisation, le point de référence est le cockpit d'un autre aéronef (par exemple escorteur). Dans d'autres modes de réalisation le point de référence peut être une porte de secours, ou un local de repos, ou un siège passager, etc.

Dans un mode de réalisation, la requête est reçue par un point d'accès sans-fil associé à une passerelle sécurisée, ladite passerelle sécurisée déterminant les droits d'accès aux données avioniques par le dispositif non-avionique. Dans un mode de réalisation, la passerelle sécurisée pour l'échange de données entre systèmes avionique et systèmes non-avionique implémente des fonctionnalités comprenant des règles de routage, une gestion des ports de communication pour autoriser ou interdire des communications et une gestion de couches protocolaires. Dans un mode de réalisation, l'étape consistant à ajuster l'envoi de données est en outre fonction des capacités d'affichage et/ou de calcul du dispositif client non-avionique. Dans un mode de réalisation, l'envoi de données est réduit ou stoppé ou augmenté ou modifié si la distance déterminée est au delà ou en deçà de seuils ou de plages de seuils prédéfinis. Dans un mode de réalisation, l'étape consistant à déterminer la distance ou la position entre le dispositif non-avionique et le cockpit de l'aéronef étant effectuée par application d'une ou de plusieurs technologies comprenant le positionnement par empreinte radio FM, par utilisation d'un réseau de balises Bluetooth BLE, par l'utilisation d'un réseaux de balise RFID, par l'utilisation d'un tapis de sol avec des réseaux de capteurs embarqués, par combinaison de technologies RFID et WLAN, par reconnaissance d'images, par ultrasons et techniques d'angle d'arrivée, par utilisation d'une centrale à inertie, et/ou de positionnement par mesure de champ magnétique ambiant. Dans un mode de réalisation, les communications sont unidirectionnelles depuis des systèmes avioniques vers le dispositif non-avionique, lesdites communications comprenant une ou plusieurs notifications selon une ou plusieurs modalités, comprenant une modalité visuelle, sonore et/ou vibratile. Dans un mode de réalisation, les communications sont bidirectionnelles entre les systèmes avioniques et le dispositif non-avionique. Dans un mode de réalisation, le dispositif non-avionique comprend une oreillette et/ou une montre connectée et/ou une paire de lunettes connectées. Dans un mode de réalisation, le procédé comprend en outre une étape consistant à envoyer des données depuis le dispositif client non-avionique vers les systèmes avioniques du cockpit, l'envoi étant sans-fil depuis des zones d'espace prédéfinies et non publiques dans l'aéronef, et/ou par l'intermédiaire de liaisons filaires non accessibles aux passagers. Dans un mode de réalisation, le procédé comprend en outre une ou plusieurs des étapes consistant à conditionner l'envoi de données à l'enregistrement préalable du dispositif non-avionique auprès du cockpit, à procéder à une étape d'authentification sécurisée et/ou biométrique, à vérifier des conditions physiologiques du porteur du dispositif non-avionique, et/ou à chiffrer les communications.

Concernant notamment *la gestion (fusion, assimilation, apprentissage, etc) des données et les modalités d'affichage* et/ou les aspects décrits ci-dessus, il est décrit un procédé pour la communication de données de type avionique à un dispositif client léger de type non-avionique, le procédé comprenant les étapes consistant à :- recevoir et afficher des données avioniques depuis une pluralité de systèmes avioniques ;- modifier les données avioniques reçues, par addition ou suppression ou fusion de données ;- afficher les données modifiées sur un ou plusieurs clients légers non-avioniques. Dans un mode de réalisation les données des systèmes avioniques sont accessibles aux clients légers non-avioniques par l'intermédiaire d'un point d'accès sans-fil associé à une passerelle sécurisée comprenant des fonctions de routage, de gestion de droit d'accès et de couches protocolaires. Dans un mode de réalisation, les communications entre les systèmes avioniques et les clients légers non-avioniques étant multiplexées. Dans un mode de réalisation, le procédé comprend en outre l'étape consistant à associer exclusivement un ou plusieurs systèmes avioniques à un ou plusieurs dispositifs non-avioniques. Dans un mode de réalisation, le procédé comprend en outre les étapes consistant à déterminer les ressources en calcul et/ou en affichage d'un client léger non-avionique, dites ressources cibles; et à ajuster l'affichage des données modifiées sur ledit client léger non-avionique en fonction desdites ressources cibles. Dans un mode de réalisation, l'étape d'affichage consiste à distribuer l'affichage sur un ou plusieurs écrans préexistants, notamment de type IFE, situés à proximité du dispositif non-avionique. Dans un mode de réalisation, le procédé comprend en outre l'étape consistant à projeter à proximité d'un porteur d'un client léger non avionique une image représentant les données avioniques modifiées sur un ou plusieurs supports ne constituant pas a priori des écrans d'affichage en déformant l'image projetée de manière à se conformer aux aspérités et/ou discontinuités des surfaces sur lesquelles l'image est projetée. Dans un mode de réalisation, le procédé comprend en outre une étape consistant à déterminer une vue subjective du porteur du dispositif non-avionique à l'aide d'une caméra portée par ledit porteur. Dans un mode de réalisation, plusieurs clients légers non-avioniques communiquent entre eux via la passerelle sécurisée. Dans un mode de réalisation, un client léger non-avionique est une oreillette connectée et/ou une montre connectée et/ou une paire de lunettes connectées.

Concernant notamment les paramètres de vol manipulés depuis un client léger, et/ou les aspects décrits ci-dessus, il est décrit un procédé pour la communication de données de type avionique à un dispositif de type non-avionique, le procédé comprenant les étapes consistant à : - recevoir un paramètre de vol depuis un client léger non-avionique;- déterminer ou recevoir des données physiologiques et/ou des données biométriques associées au porteur du client léger non-avionique ;- conditionner l'insertion du paramètre de vol dans un système avionique à la satisfaction de conditions prédéfinies portant sur les données physiologiques et/ou biométriques. Dans un mode de réalisation, le procédé comprend en outre une étape consistant à déterminer la distance entre le dispositif non-avionique et le cockpit de l'aéronef; et les conditions prédéfinies portant en outre sur ladite distance déterminée. Dans un mode de réalisation, le procédé comprend en outre une étape de vérification d'intégrité du message de commande/paramètre de vol transmis et/ou une étape de chiffrement. Dans un mode de réalisation, les conditions physiologiques sont en rapport avec un ou plusieurs paramètres comprenant le suivi du regard, le suivi du mouvements des yeux, les fixations du regard, le taux de cortisol, le rythme cardiaque, la variabilité de ce rythme cardiaque, un ou plusieurs marqueurs de l'activité du système parasympathique, le rythme respiratoire, la température de la peau, le niveau de sudation, la conductivité de la peau, la dilatation pupillaire, un signal d'électrocardiographie, un signal d'électroencéphalographie, et un signal de magnétoencéphalographie. Dans un mode de réalisation, le procédé comprend en outre une étape consistant à associer la commande de vol reçue à une pluralité de requêtes élémentaires communiquées vers plusieurs équipements avioniques, lesdites requêtes étant notamment associées à une ou plusieurs interfaces de programmation APIs. Dans un mode de réalisation, la commande de vol (ou donnée ou paramètre de vol) est communiquée par voie audio et traitée par reconnaissance vocale. Dans un mode de réalisation, le procédé comprend en outre une étape consistant à diffuser un message depuis un système avionique vers le client léger non-avionique. Dans un mode de réalisation, le message est restitué selon une ou plusieurs modalités de forme choisies parmi une vibration, un son, une image ou une vidéo. Dans un mode de réalisation, le message est une notification configurable ou désactivable ou acquittable. Dans un mode de réalisation, un client léger non-avionique est une oreillette connectée et/ou une montre connectée et/ou un ordinateur porté et/ou un bracelet.

Dans un mode de réalisation, un système avionique est associé avec un taux de panne physique inférieur et une vérification logique supérieure à ceux d'un système de type non-avionique.

Dans un mode de réalisation, un système avionique est associé avec une exhaustivité des tests et/ou de vérifications supérieure à ceux d'un système de type non-avionique.

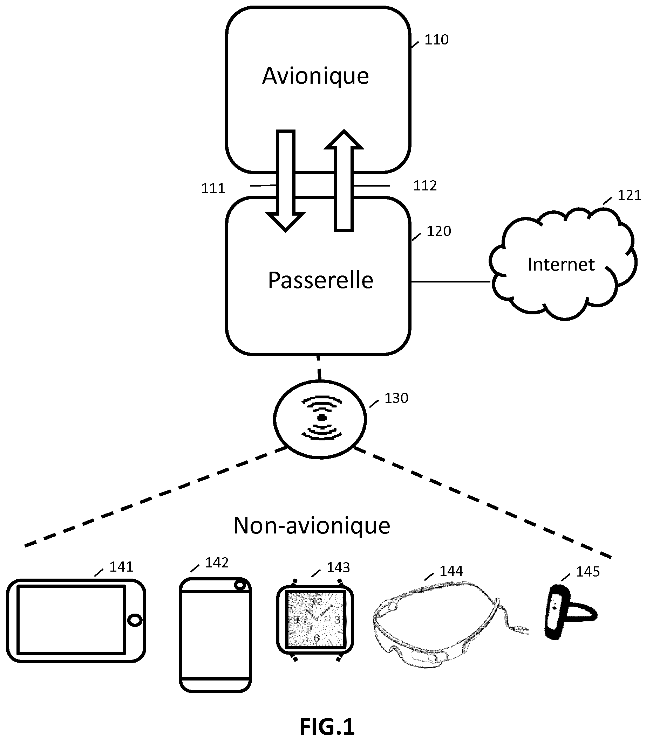

La figure 1 illustre certains aspects de l'architecture générale des modes de réalisation de l'invention.

Les données du cockpit de type avionique 110 sont communiquées à une passerelle sécurisée 120, laquelle dessert (directement ou indirectement via des sous systèmes non- avionique) un ou plusieurs clients légers de type tablette (ou ordinateur portable) 141, téléphone portable 142, montre connectée 143, lunettes connectées 144, oreillette connectée 145 (e.g. Bluetooth ou Wifi, externe, implantée, etc) ou selon d'autres systèmes d'affichage et/ou de restitution d'informations (non représentés), lesquels comprennent notamment un combiné casque-micro (un microphone peuvent notamment opérer par conduction osseuse), un pico-projecteur, etc. De manière encore plus générale, un client léger peut être un ordinateur porté ou portable (« wearable computer » en anglais), sous n'importe quelle forme (bracelet pico-projecteur, implant rétinien, e-textile, etc).

Dans un mode de réalisation, un client léger exécute une application mobile. Dans la suite de ce document, on appellera « application locale » l'application logicielle qui s'exécute sur l'appareil connecté.

L'application locale sur l'appareil est connectée au monde avionique (par exemple en wifi) via une passerelle sécurisée, elle-même connectée aux différentes fonctions ou calculateurs embarqués (via le réseau avionique, par exemple en AFDX), ainsi qu'à d'autres sources de données du monde ouvert 121. Conceptuellement il n'y a pas de limite au nombre d'utilisateurs connectés. De plus les utilisateurs peuvent recouvrir des profils divers (pilotes, PNC). Les applications locales s'adaptent à la fois au type de l'appareil connecté (ressources et capacités d'affichage disponibles) et au profil de l'utilisateur (sélection de données pertinentes en fonction du type de la mission de l'utilisateur).

Opérationnellement le nombre d'utilisateurs peut toutefois être limité du fait des capacités du réseau et des contraintes de sécurité à gérer.

Dans un mode de réalisation facultatif, une application locale peut s'appuyer sur une application tierce qui s'exécute en complément des applications locales. Cette application tierce peut par exemple faire office de proxy pour les fonctions locales qui interagissent avec les fonctions avioniques à travers l'application tierce.

Cette application tierce peut être hébergée par la passerelle sécurisée elle-même ou par une machine tierce non nécessairement mobile, située dans le monde ouvert et qui se connecte à la passerelle via le point d'accès sans fil (par exemple wifi). Dans la suite de ce document, on parlera de l'« application centrale » pour désigner cette application tierce.

Dans un mode de réalisation le procédé selon l'invention permet des interactions entre clients légers et fonctions avioniques (e.g. passage de commandes intégrées à haut niveau d'abstraction)

### Passerelle sécurisée

Cette passerelle permet la communication entre le « monde ouvert » et le monde avionique. Généralement, cette passerelle peut être en charge des fonctions suivantes :
- fonction de routage (la passerelle dispose d'une fonction de configuration qui permet d'autoriser ou d'interdire des connexions (111 et/ou 112) entre les équipements du monde avionique et ceux du monde ouvert via des règles de routage) ;
- fonction de filtrage de sécurité : la passerelle dispose d'une fonction de configuration qui permet d'autoriser ou d'interdire la communication entre équipements, par exemple via des ports spécifiés (fonctions de type pare-feu ou *firewall en anglais*), notamment dans le sens du monde ouvert vers le monde avionique 112 qui requiert un haut niveau d'intégrité des données ;
- fonction de gestion des couches protocolaires : la passerelle dispose de fonctions de transformations des protocoles spécifiques de l'avionique en protocoles du monde ouvert (par exemple TCP/IP ou UDP/IP) et vice-versa.

De manière plus générale, les systèmes avioniques et non-avioniques interagissent par l'entremise d'un organe de régulation, par exemple la passerelle sécurisée. Les principes de la régulation des échanges entre les systèmes avioniques et systèmes de type non-avionique peuvent être divers. Les différentes modalités peuvent prendre en compte (i.e. moduler directement ou indirectement) un ou plusieurs des paramètres suivants : a) la directionalité des échanges (unidirectionnelle et/ou bidirectionnelle, statique et invariante au cours du temps, ou évolutive, par exemple suivant le contexte de vol ou des règles prédéfinies ; b) la forme des données échangées (e.g. format de données, type de protocoles, traduction/pontage, etc). Des algorithmes de compression ou de transcodage peuvent être utilisés. c) le fond, i.e. la nature ou la qualité des objets communiqués ; ces objets peuvent être filtrés, censurés, adaptés en fonction des besoins de l'avionique, etc. d) la quantité (ou le volume) des données échangées. L'avionique ne devant généralement pas être sur-sollicitée (gestion des ressources et propagation d'erreurs), un suivi des volumes et un ajustement de ces derniers est avantageux. e) des privilèges ou des priorités associés aux données et/ou aux systèmes matériels. L'allocation des rôles, privilèges ou priorités pourra être prédéfinie ou dynamique. Les architectures ou modèles peuvent être variés: systèmes maitres/esclaves, évolutifs ou non, réseaux de pairs-à-pairs, etc.

### Accès sans fil

Dans un mode de réalisation de l'invention, le système pour l'accès aux données hors du cockpit comprend un point d'accès sans fil 130 (Wifi ou Li-Fi ou autre ; le Li-Fi pour *Light Fidelity* est une technologie de communication sans fil basée sur l'utilisation de la lumière visible, de longueur d'onde comprise entre 480 nm et 650 nm) Ce point d'accès est utilisé par les équipements du monde ouvert pour se connecter entre eux ou pour se connecter à la passerelle afin de communiquer avec les équipements avioniques. Il fournit de plus un accès à l'internet.

Ce point d'accès sans fil peut être intégré directement à la passerelle ou se situer dans le monde ouvert. Dans tous les cas, la passerelle est reliée au point d'accès sans fil.

Comme indiqué précédemment, l'invention envisage la possibilité que la passerelle puisse être modifiée afin d'héberger tout ou partie de l'application centrale. Les avantages et inconvénients de cette possibilité sont précisés plus loin.

Suivant le niveau de sécurité offert par la passerelle, sa configuration (notamment le sens unidirectionnel 111 ou bidirectionnel (111,112) des communications entre le monde avionique et le monde ouvert) et la possibilité de lui allouer des fonctions supplémentaires (ici pour héberger tout ou partie de l'application centrale), différents modes de réalisation sont envisageables.

Les procédés et les systèmes selon l'invention peuvent notamment permettre la mise à disposition pour l'utilisateur d'un ensemble de fonctions qui vont lui permettre d'accéder en permanence, et depuis n'importe où dans l'avion, à des données issues du monde avionique (et combinées ou pas avec des données du monde ouvert). Ces fonctions permettent aussi d'interagir avec les systèmes qui fournissent les données pour leur envoyer des paramètres de vol. Enfin l'invention fournit aussi un ensemble de fonctions techniques qui vont améliorer l'ergonomie de la solution, par exemple en fonction du type de l'appareil connecté ou du profil de l'utilisateur.

A partir de la liste de ces fonctions, l'invention propose différentes alternatives possibles de projection de ces fonctions sur différents équipements : (a) sur l'appareil léger connecté situé dans le monde ouvert ; (b) sur une machine tierce (non nécessairement mobile située dans le monde ouvert) ; (c) sur la passerelle sécurisée située dans le monde avionique.

Selon certains modes de réalisation, le procédé comprend une étape consistant à déterminer les capacités de calcul et/ou les capacités d'affichage d'un ou de plusieurs équipements ou appareils. Cette analyse peut être conduite sur le périmètre d'un seul client léger, mais également sur une pluralité d'appareils accessibles et situés à proximité. Par exemple, la distribution de l'affichage permettra d'utiliser la surface d'affichage de la montre connectée et d'un écran IFE à proximité, sur demande (par « glisser-déposé) depuis la montre connectée du pilote. De même, une paire de lunettes connectée peut travailler de concert avec les écrans affichés dans le cockpit ou un pico-projecteur situé tout à l'arrière de la cabine. Les différentes combinaisons peuvent être notées (e.g. scores, notes, pondérations, triées, classées, etc). Dans un mode de réalisation, le(s) client(s) cibles peuvent être déterminés en faisant abstraction des capacités réelles, puis des hypothèses préférentielles de projection peuvent être déterminées par rapport au rôle de la fonction, sachant qu'in *fine* la nature de l'appareil connecté peut encore contraindre les solutions possibles.

Les applications locales peuvent s'appuyer sur une application centrale qui s'exécute en complément des applications locales. L'application centrale peut faire office de proxy pour les fonctions locales qui interagissent avec les fonctions avioniques à travers l'application centrale. L'application centrale est optionnelle, toutefois sa présence en renfort des applications locales présente certains avantages, notamment la mutualisation de fonctions accessibles depuis n'importe quel appareil connecté ; le fait de pallier l'absence ou le manque de ressources locales (i.e. au niveau de l'appareil connecté) ; le fait de permettre de récupérer des données du monde avionique ou du monde ouvert en temps masqué pour les applications locales (amélioration des temps de réponses) ; l'apport d'une garantie de cohérence des informations entre les différents utilisateurs (serveur centralisé de données et de requêtes) ; mise en oeuvre de fonctions d'administration centralisées sur l'ensemble des utilisateurs (par exemple la gestion de profils, la gestion d'autorisations par groupes d'utilisateurs).

L'application centrale peut être hébergée par la passerelle ou par une machine tierce (non nécessairement mobile) située dans le monde ouvert et qui se connecte à la passerelle.

Dans certains modes de réalisation, une partie des fonctions sont allouées à la machine tierce et une autre partie à la passerelle (application centrale distribuée ou répartie entre la machine tierce et la passerelle).

Dans un mode de réalisation, une application locale envoie une requête de consultation vers un équipement avionique à travers une application centrale hébergée par une machine tierce (non représentée). Les échanges peuvent être ou comprendre les échanges suivants :
1. Emission d'une requête vers un équipement avionique par l'utilisateur depuis l'application locale qui s'exécute sur l'appareil connecté (e.g. 141, 142, 143, 144, etc) ; l'application locale émet cette requête vers l'application centrale via le point d'accès sans fil 130 ;
2. Le point d'accès sans fil 130 retransmet la requête vers l'application centrale qui s'exécute sur une machine tierce ;
3. L'application centrale se connecte au point d'accès sans fil 130 dans le but de transmettre la requête vers les équipements avioniques concernés via la passerelle ;
4. Le point d'accès sans fil 130 reçoit la requête en provenance de l'application centrale puis la transmet aux fonctions de la passerelle sécurisée 120 qui gère les accès vers les équipements avioniques;
5. La passerelle sécurisée 120 adresse un ou plusieurs équipements avioniques concernés 110 avec la requête reçue ;
6. Un ou plusieurs équipements renvoient vers la passerelle sécurisée 120 le retour d'exécution de la requête ;
7. La passerelle sécurisée 120 retransmet ce retour 110 vers le point d'accès sans fil 130 ;
8. Le point d'accès sans fil 120 retransmet à son tour le retour vers l'application centrale sur la machine tierce ;
9. L'application centrale (après éventuellement un post-traitement) rappelle le point d'accès sans fil 130 qui retransmet le retour consolidé de la requête vers l'application locale qui s'exécute sur l'appareil connecté (e.g. 141, 142, 143, 144, etc)

### Diffusion de données du monde avionique

La passerelle sécurisée 120 selon l'invention est un équipement intermédiaire qui s'insère dans le monde avionique et qui est connecté au réseau avionique. Cette passerelle héberge les fonctions de routage et de sécurité nécessaires qui permettent de diffuser les données avioniques vers le monde ouvert à travers un point d'accès sans fil tout en préservant la sécurité des données avioniques. Elle permet la communication de données (111, 130) depuis les fonctions et équipements avioniques vers le monde ouvert. Les fonctions et équipements avioniques communiquent entre eux à travers un réseau propre (par exemple de type AFDX) pour lequel les exigences de certification sont élevées. Ce réseau est en général configuré de manière statique afin de rendre son comportement déterministe. La passerelle fournit les fonctionnalités permettant au réseau avionique de préserver ses caractéristiques avant de permettre la diffusion de données avioniques vers le monde ouvert (ou vice-versa).

Dans un mode de réalisation, la fonction de routage hébergée par la passerelle spécifie les connexions «point à point» autorisées entre un équipement ou une fonction avionique et un équipement du monde ouvert. Les règles de routage peuvent être évolutives (e.g. dès qu'une nouvelle connexion est requise entre deux équipements). Dans un mode de réalisation, l'émission de données en mode broadcast par un équipement avionique permet à n'importe quel équipement connecté du monde ouvert de recevoir ces données sans qu'il soit nécessaire de modifier les règles de routage.

Dans un mode de réalisation, la passerelle sécurisée 120 est connectée à Internet 121 et le mode ouvert. Elle comprend alors les mécanismes permettant d'éviter l'injection de données dans les systèmes avioniques.

Dans un mode de réalisation, le point d'accès sans fil 130 est connecté à l'internet 121 et assure le rôle de fournisseur des données du monde ouvert en provenance de l'internet, aux équipements connectés.

### Routage de requêtes non-avioniaues vers les équipements du monde avionique

Cette fonction est la fonction réciproque de la fonction précédente. Elle fournit le routage vers les fonctions et équipements avioniques des demandes ou requêtes de consultation émises par les équipements du monde ouvert.

Dans un mode de réalisation, la passerelle sécurisée héberge les fonctions de routage et de sécurité qui permettent de router les paramètres de vol émis par les équipements du monde ouvert et reçus via le point d'accès sans fil, vers les fonctions et équipements du monde avionique, tout en préservant la sécurité.

Dans un mode de réalisation, la passerelle sécurisée peut déterminer les fonctions de routage, par exemple en spécifiant les connexions « point à point » autorisées entre un équipement du monde ouvert et un équipement ou une fonction du monde avionique. Dans un mode de réalisation, la passerelle sécurisée peut aussi déterminer et enclencher un mode de diffusion broadcast ou multicast.

### Diffusion multiplexée de données

Dans un mode de réalisation, la diffusion de données est effectuée de manière multiplexée. Avantageusement, cette diffusion multiplexée permet de remédier aux limitations qui peuvent exister du fait du grand nombre de connexions possibles entre une fonction (aspect logique) ou un équipement avionique (aspect physique) et des équipements du monde ouvert. En effet les fonctions et équipements avioniques ne permettent pas de gérer un nombre infini de (demandes de) connexions.

Dans un mode de réalisation, un seul serveur de données existe entre les clients légers et les systèmes avionique. Ce schéma permet de gérer les contraintes, de les lisser, et/ou de les ordonner dans le temps (e.g. priorités ou criticité variables, droits d'accès, etc). Ce mode multiplexé peut permettre de connecter virtuellement un grand nombre d'équipements du monde ouvert à un même équipement avionique). En d'autres termes, les ressources « rares » sont celles du monde avionique et il convient de les solliciter à bon escient (e.g. systèmes de cache, etc). Du fait de son rôle de serveur, cette fonction peut avantageusement être allouée à l'application centrale et/ou à une machine tierce afin de ne pas modifier le périmètre avionique.

### Multiplexage des requêtes

A l'instar de la diffusion, le procédé selon l'invention peut comprendre un multiplexage des requêtes ; de façon à remédier aux limitations qui peuvent exister en matière du nombre de connexions possibles entre une fonction ou un équipement avionique et des équipements du monde ouvert. Dans un mode de réalisation, un serveur de requête reçoit les requêtes des différents équipements connectés du monde ouvert, ce serveur étant le seul à être connecté directement à la fonction ou équipement avionique qui traite la requête. Ce schéma permet de déporter les contraintes de connexions sur la fonction plutôt que sur le fournisseur de services. Dans un mode de réalisation, des files d'attente ou d'enfichage peuvent être gérées aux fins de distribuer les requêtes aux équipements et/ou les fonctions avioniques, notamment quand ces derniers deviennent disponibles. De cette manière les équipements et fonctions physiques sont vus comme des équipements et fonctions logiques depuis les applications locales. Ce mode de réalisation peut donc permettre de connecter un grand nombre d'équipements du monde ouvert à un même équipement avionique.

### Répartition de charge (« load-balancing » en anglais

À côté du multiplexage des diffusions de données et/ou des requêtes, différents mécanismes de répartition de charge peuvent être implémentés. Le procédé selon l'invention peut ainsi comprendre une étape consistant à déterminer vers quelle fonction ou vers quel(s) équipement(s) avioniques il convient d'orienter une requête utilisateur, notamment afin d'optimiser l'utilisation des ressources du système global. Cette répartition de charge suppose que plusieurs fonctions ou équipements avioniques sont à même de satisfaire une même requête utilisateur.

L'optimisation de la répartition de charge peut concerner les temps de réponse et ou la répartition de la charge de calcul entre les différents équipements.

Ce mode de réalisation reste entièrement facultatif. L'avantage le cas échéant est généralement de répartir les charges de calcul.

Dans un mode de réalisation, la répartition de charge est effectuée par l'application centrale et/ou une machine tierce qui « virtualise » les équipements et fonctions physiques pour les applications locales.

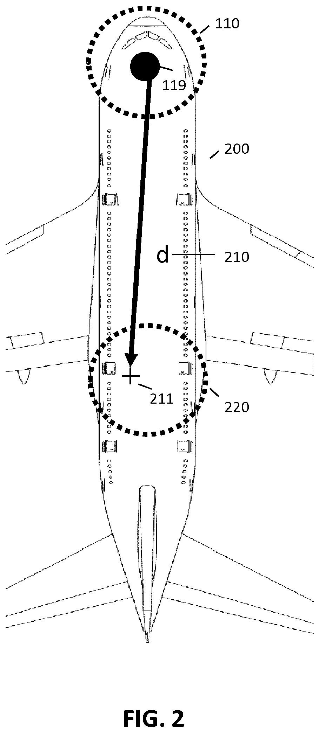

La figure 2 illustre un mode de réalisation de l'invention, dans lequel la distance dans l'espace entre le cockpit et un client léger est manipulée pour l'accès aux données avioniques.

Les équipements de type avionique 110 de l'aéronef 200 sont essentiellement situés dans le cockpit 119 (e.g. FMS). Le personnel navigant (PNC ou pilote, etc) peut se déplacer dans l'avion. A un instant donné du temps, un client léger se trouve à une distance d 210 du cockpit, dans une zone d'espace 220. Cette donnée physique est mesurable, de diverses manières (décrites ci-après). La « distance » est indicative de la « position » 211, modulo les informations topologiques (sièges et couloirs de l'avion). Pour une distance donnée, un pilote (par exemple) se trouve dans le couloir gauche ou droit de l'avion. Quand les technologies de positionnement sont plus précises, la position quasiment exacte peut être déterminée.

Cette valeur de distance 210 peut conditionner l'accès aux données avioniques (afin d'émettre et/ou de recevoir). Des plages de distance peuvent en effet réguler ou moduler les accès. Dans un mode de réalisation, afin de recevoir et/ou de recevoir de l'information, il peut être exigé de se tenir dans certaines zones de l'avion (sécurité « cachée »). Dans un mode de réalisation, si un utilisateur est suffisamment proche du cockpit, sous un seuil de distance prédéfini, les paramètres de vol modifiables à distance peuvent être désactivés. Dans un mode de réalisation, la quantité et/ou la « qualité » des informations (e.g. criticité, tags, teneur, etc) est fonction de la distance 210. Dans un mode de réalisation, le flux (quantitatif et/ou qualitatif) peut être dégressif en fonction de la distance. Dans un mode de réalisation, le flux peut être inversement proportionnel à la distance (un pilote coincé à l'arrière de l'appareil en cas de détournement peut disposer de capacités étendues)

L'étape de détermination de la distance peut être effectuée de diverses manières, éventuellement combinée (pondérée) entre elles. Un « système de positionnement en intérieur » ou « système de géolocalisation en intérieur » permet de trouver la position d'objets ou de personnes dans un espace interne à une structure. Incidemment, les itinéraires peuvent être surveillés (« monitoring » en anglais) et peuvent servir de conditions pour les accès aux données. Des modèles topologiques peuvent représenter les propriétés des connectivités (pièces, couloirs, etc) dans un espace intérieur

Différentes technologies de position en intérieur peuvent être utilisées, éventuellement en combinaison: positionnement par empreinte radio FM, réseaux de balises Bluetooth BLE, réseaux de balise RFID, tapis de sol avec des réseaux de capteurs, combinaison des technologies RFID et WLAN, reconnaissance d'images, par ultrasons et techniques d'angle d'arrivée, utilisation de signaux de localisation d'une centrale à inertie, positionnement par mesure de champ magnétique ambiant, etc.

Dans les détails, l'approche par Radio FM ou localisation par "empreinte radio" ("*fingerprinting signal*" en anglais) comprend des étapes d'étalonnage et de localisation. Les ondes FM présentent une efficacité énergétique supérieure au signal Wifi (entre 2 et 6 fois supérieure en matière d'autonomie). Un ou plusieurs réseaux de balises BLE ou RFID ou autres peuvent permettre de positionner un client léger. La combinaison des technologies RFID et WLAN consiste à combiner des identifiants de tags RFID à la localisation ainsi qu'aux informations topologiques afin de déterminer la position et de prévoir le prochain sous-réseau d'un noeud mobile (exploitation des capacités de coordination fournies par le WLAN). Les approches fondées sur la vision par ordinateur sont évolutives et peu onéreuses (tracking d'objet, etc). Le positionnement par ultrasons et techniques d'angle d'arrivée consiste à déterminer le temps de propagation d'un signal pour déterminer une position. Une centrale à inertie ou un odomètre ou d'autres types de capteurs peuvent être utilisés en complément des techniques mentionnées (filtre de Kalman étendu pour l'intégration de données en temps réel).

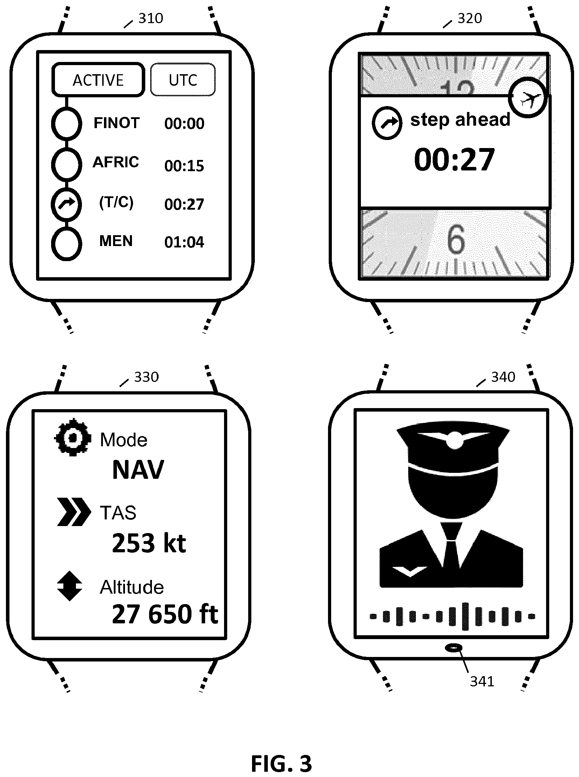

La figure 3 illustre différent exemples d'interface utilisateurs, dans le cas d'un mode de réalisation de l'invention implémentée sur une montre connectée.

### Notifications

### Avantageusement, une ou plusieurs notifications peuvent être déterminées puis communiquées à un ou plusieurs utilisateurs/

Une notification permet d'avertir un utilisateur de l'occurrence d'un évènement donné sans que ce dernier ait à consulter lui-même régulièrement les données du système pour évaluer l'occurrence de cet évènement (fonction de « réveil » ou de « push »). Les notifications permettent d'éviter à l'utilisateur d'avoir à consulter périodiquement les données de base sur lesquelles est basé l'événement, ainsi que d'avoir à croiser ces données de base pour évaluer l'occurrence de l'événement. Les notifications permettent donc de libérer du temps pour l'utilisateur afin qu'il puisse se concentrer sur d'autres tâches. Elles peuvent permettre d'éviter les erreurs humaines. Elles peuvent également permettre de réduire le stress de l'utilisateur n'ayant plus à redouter de manquer l'occurrence de l'événement.

Un évènement considéré par une notification peut concerner une donnée fournie par un équipement avionique (par exemple le système de gestion de vol FMS peut fournir l'information selon laquelle l'avion commence sa descente vers l'aéroport de destination) ou correspondre à une information résultant de la combinaison de données fournies par plusieurs systèmes (par exemple la traversée prochaine d'une zone de turbulence, cette information résultant du croisement de la trajectoire de l'avion fournie par le FMS et des données météorologiques fournies par un serveur accessible depuis le monde ouvert).

L'interface 310 illustre un exemple d'interface ou écran, lequel fournit des points de passage ou point de plan de vol. L'exemple 320 illustre une notification selon laquelle il est rappelé au pilote qu'un changement de niveau de plan de vol est prévu dans le futur (dans 27 minutes). L'exemple 330 illustre une vue synthétique de la situation de l'aéronef (par exemple comprenant l'altitude courante ou « *flight level* » en anglais). Dans certains modes de réalisation de l'invention, par exemple 340, la montre connectée comprend une caméra 341 permettant notamment des échanges vidéos entre le pilote et le copilote.

Dans un mode de réalisation, les notifications sont configurables. Un utilisateur peut en effet souhaiter s'abonner à des notifications prévues par défaut et/ou programmer ses propres notifications (par exemple en sélectionnant les champs de données nécessaires et en spécifiant les règles de combinaison de ces données pour évaluer les occurrences d'un évènement d'intérêt).

Dans un mode de réalisation, le pilote peut également activer ou désactiver des notifications, par exemple en fonction des moments de sa mission opérationnelle.

Dans un mode de réalisation, le pilote peut déterminer ou configurer le niveau de criticités associées aux différentes notifications (par exemple entre informations générales, alertes, messages d'urgences, etc).

Les notifications peuvent être émises sous diverses formes ou selon différentes modalités.

Dans un mode de réalisation une notification est émise sous forme visuelle. Par exemple, l'affichage peut être virtuellement affiché dans les lunettes connectées ou via un visiocasque porté par le pilote.

Dans un mode de réalisation, une notification est émise sous forme sonore. Par exemple le pilote peut porter une oreillette ou un casque audio ou un combiné casque micro.

Dans un mode de réalisation, une notification peut être émise sous forme vibratoire. Par exemple le pilote peut porter une montre connectée comprenant un vibreur.

Dans un mode de réalisation, une notification est émise sous forme multimodale i.e. en combinant une ou plusieurs des modalités comprenant un affichage, une émission sonore, une vibration ou tout retour haptique.

Avantageusement, selon les modes de réalisation de l'invention, une notification peut être strictement personnelle (e.g. codage du nombre de vibrations, affichages virtuels et personnels, émission d'un son dans le casque etc.). Dans certains modes de réalisation de l'invention une notification peut être publique (par exemple affichage simultané sur les écrans IFE).

Pour être strictement personnelles, certaines notifications peuvent par exemple êtres codées en nombre de vibrations, via des affichages internes et subjectifs dans un visiocasque, etc).

Par ailleurs, au-delà de l'aspect public ou privé d'une notification, l'attention de l'utilisateur peut être captée ou capturée de manière sûre (la probabilité que le message parvienne bien à destination est forte). Dans un mode de réalisation de l'invention, un système d'acquittement des notifications permet d'obtenir l'assurance que le message est correctement délivré (e.g. tapotement sur la montre, acquittement par glissement d'une icône, entrée d'un code PIN, clignement d'oeil, etc.)

La gestion des notifications peut être implémentée sur l'application centrale et sur une machine tierce (mutualisation de la gestion d'événements intéressants plusieurs utilisateurs). Ce mode de réalisation permet en outre d'alléger la charge réseau, en évitant à chaque utilisateur d'accéder indépendamment aux équipements avioniques.

Dans un mode de réalisation, la gestion des notifications est implémentée de manière locale (application locale) par exemple si la condition ou l'événement à surveiller est spécifique un utilisateur précis.

Par ailleurs, les notifications peuvent être régulées de différentes manières. Une notification peut être communiquée de manière générale à tous les clients légers, ou de manière spécifique (c'est-à-dire à une sélection de clients légers). L'application de règles locales peut également moduler la survenue des notifications locales. Ces règles locales peuvent inhiber ou ajouter ou supprimer ou substituer une notification par une ou plusieurs autres notifications.

### Fusion de données et gestion de l'affichage

Les données avioniques peuvent être dispersées. Il peut être avantageux de les modifier (ajout, suppression, substitution, croisement avec des données du monde ouvert etc) avant de les redistribuer selon différentes modalités.

### Des données avioniques dispersées

Les données d'une fonction (ou d'un sous-système avionique) sont en général accessibles par l'intermédiaire d'une seule interface : celle de la fonction ou du sous-système avionique en question. En revanche, un utilisateur peut avoir besoin de consulter plusieurs fonctions ou sous-systèmes avioniques (et donc autant d'interfaces) pour apprécier globalement une situation opérationnelle. Les modes de réalisation de l'invention permettent de gérer ces données éparses

Physiquement, les interfaces peuvent être localisées à différents endroits (dispersées) dans le cockpit et/ou la cabine (le reste de l'avion hors cockpit). Pour le pilote, la nécessité de consulter l'ensemble des interfaces séparément peut nuire à la réactivité parfois nécessaire pour prendre une décision opérationnelle et demande une grande concentration au pilote. En outre, en supposant que le pilote dispose de l'ensemble des données nécessaires, il a encore besoin de croiser ces données, de les filtrer, de les consolider, de les synthétiser, avant de disposer de données opérationnelles pour la prise de décision. Cette activité de traitement de données ralentit là aussi sa réactivité à la prise de décision. De plus elle est une source d'erreur humaine.

### Distribution d'affichage

Dans un mode de réalisation, l'affichage selon l'invention peut être « distribué » (sur la forme) sur un ou plusieurs clients légers et des étapes de fusion et/ou de réduction d'informations (sur le fond) peuvent être mises en oeuvre, dans le but d'assister le pilote dans sa prise de décision.

Les modes de réalisation de l'invention ne limitent pas *a priori* la nature des appareils connectés à utiliser. En revanche les capacités de ces derniers, notamment en termes de possibilités d'affichage et de puissance de calcul propre, permettent d'ajuster les affichages et d'optimiser l'utilisation des ressources disponibles. L'application locale peut avoir la capacité de détecter la nature de l'appareil sur lequel elle s'exécute afin de déterminer les ressources disponibles, puis de s'auto-adapter en activant automatiquement la configuration offrant le plus grand nombre de fonctions possibles et permettant l'affichage le plus riche qui soit. Par exemple, au démarrage, l'application locale peut déterminer le type d'appareil connecté puis les caractéristiques propres à cet appareil (par exemple les capacités d'affichage et la puissance CPU). L'application locale peut ensuite sélectionner automatiquement la configuration optimale exécutable sur cet appareil (ou un ensemble d'appareils)

### Réduction, augmentation et fusion de données

La fusion de données peut se combiner avec la diffusion multiplexée de données.

Ce mode de réalisation vise à aider les utilisateurs pour leurs prises de décisions opérationnelles. En effet les données brutes fournies par les différents équipements avioniques ou du monde ouvert ne sont pas nécessairement directement exploitables dans leur état brut pour fournir les informations nécessaires à l'utilisateur dans sa prise de décision. Souvent une information utile pour l'opérateur est le résultat du croisement de plusieurs données élémentaires fournies par un nombre plus ou moins important d'équipements.

La fusion de données consiste notamment à prendre à sa charge ces opérations de sélection, de croisement, de combinaison, de consolidation, de filtrage etc. qui sont aujourd'hui effectuées par les utilisateurs eux-mêmes (avec un risque d'erreur non négligeable). La fusion d'informations dépasse l'automatisation de l'acte mental, puisqu'en quantité, vitesse et vitesse d'analyse les capacités machines permettent aujourd'hui une redéfinition de la coopération homme-machine.

Dans les modes de réalisation dans lesquels cette fusion d'information est implémentée sur l'application centrale, elle évite aux applications locales de faire de multiples accès aux différents systèmes contributeurs, chacun de leur côté, ce qui contribue à alléger la charge réseau par mutualisation des accès. Enfin cette fusion porte la responsabilité de la garantie de la cohérence des données fusionnées vis-à-vis des applications locales.

Dans un mode de réalisation, la fusion d'informations peut être effectuée sur l'application centrale et sur une machine tierce, ce qui lui confère l'avantage de mutualiser le besoin pour plusieurs appareils connectés. Dans un mode de réalisation, la fusion d'informations peut être implémentée sur une application locale si l'application centrale n'existe pas.

De manière optionnelle, l'affichage sur un ou plusieurs clients légers portés ou environnants (par rapport à l'utilisateur) peut comprendre une ou plusieurs caméras d'acquisition d'images. Une caméra peut être fish-eye, stéréoscopique, ou autre. Ce retour d'images permet de nombreux développements avantageux de l'invention. Un appareil photo ou une caméra vidéo portée peuvent permettre de capturer au moins une partie de l'ensemble des informations visuelles affichées à destination du pilote (avantageusement, ce retour vidéo pourra être placé sur une visière tête haute, des smartglasses ou tout autre équipement porté par le pilote, de manière à capturer la vue subjective du pilote). Par analyse d'image (effectuée de manière régulière fixe ou de manière continue dans le cas d'une capture vidéo), la vue subjective du pilote peut être analysée et modifiée ou corrigée, en fonction de critères prédéfinis et/ou selon des objectifs prédéfinis.

Par exemple, dans un mode de réalisation, la densité visuelle des informations qui sont affichées peut être détermine ou estimée. Par exemple, cette densité peut être estimée selon différentes sous-parties d'images et des ajustements d'affichage peuvent être déterminés de manière dynamique. Par exemple, dans le cas où un écran d'affichage deviendrait trop « encombré » (quantité de texte ou de symboles graphiques en excès par rapport à un ou plusieurs seuils prédéfinis), les informations les moins prioritaires peuvent être « réduites » ou « condensées » ou « synthétisées » sous forme de repères ou de symboles. Inversement, si la densité d'information affichée le permet, des informations réduites ou condensées ou synthétisées peuvent être développées ou détaillées ou étendues ou agrandies (ou déplacées vers des appareils d'affichage à proximité.

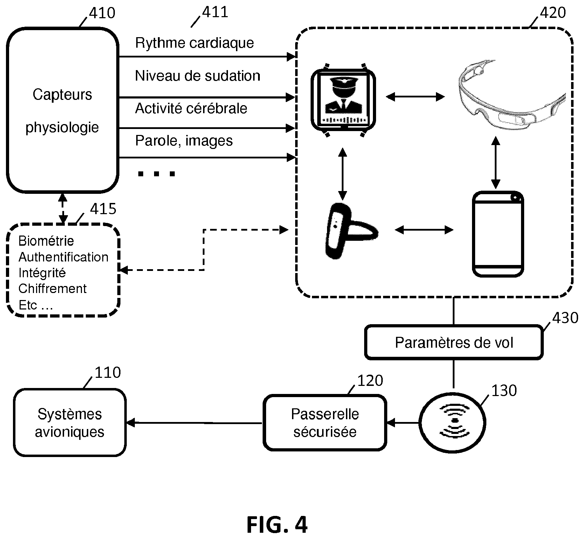

La figure 4 illustre l'envoi d'un paramètre de vol depuis un ou plusieurs clients légers à l'avionique via la passerelle sécurisée et le point d'accès.

Dans un mode de réalisation spécifique, le procédé comprend une étape consistant à communiquer des paramètres de vol 430 depuis un ou plusieurs clients légers (e.g. une montre connectée, des lunettes connectées, etc) 420 puis à injecter ces paramètres de vol via la passerelle sécurisée 120 et le point d'accès 130 dans les systèmes avioniques 110.

Les paramètres de vol (ou données de vol) peuvent prendre des formes différentes (e.g. plan de vol, instruction, contrainte, données de performances, etc...). Ces paramètres de vol alimentent l'avionique qui à son tour générera des commandes de vol concrètes (e.g. actions sur ou vers les gouvernes, etc.)

Cette possibilité de prendre des décisions via un client léger porté par le pilote ou le copilote est révolutionnaire et va à l'encontre de nombre de préjugés ou d'habitudes métier en avionique.

Des étapes optionnelles 415 comprennent une ou plusieurs étapes parmi les étapes comprenant une étape de vérification biométrique, une étape d'authentification, une étape de vérification d'intégrité du message de commande ou de paramètre de vol transmis, une étape de chiffrement, etc. Dans un mode de réalisation, un plusieurs capteurs physiologiques 410 et/ou biométriques 415 conditionnent la possibilité d'émettre des paramètres de vol 430 ou bien même la recevabilité d'un paramètre de vol.

En effet, pour être pris en compte, un paramètre de vol doit émaner d'une personne connue ou identifiée, opérant dans des conditions normales. Le fait de porter sur soi le client léger constitue en soi une première garantie d'identification, mais des tests biométriques peuvent venir confirmer (ou infirmer) cette hypothèse. Ensuite, des tests sur les conditions physiologiques (e.g. stress extrême, incertitudes ou anomalies quant à l'étant physique et/ou cognitif) peuvent moduler l'envoi de paramètres (depuis l'interdiction, l'inhibition jusqu'à la recevabilité sous conditions etc). Par ailleurs des conditions de distance et/ou de position peuvent également être prises en compte.

Dans un mode de réalisation de l'invention, un client léger comprend ou accède à un ou plusieurs capteurs physiologiques, configuré pour mesurer différents paramètres physiologiques du pilote. Par exemple le rythme cardiaque du pilote peut être mesuré par une montre connectée (ou par des oreillettes, « earbuds » en anglais) et optionnellement conditionner l'entrée d'un paramètre de vol. Par exemple, au-delà ou en deçà de seuils ou de plages de seuils prédéfinis des paramètres de vol peuvent être rejetés. Bien d'autres paramètres physiologiques peuvent être déterminés ou mesurés, et pris en compte dans la gestion de l'affichage et/ou de l'accessibilité de paramètres de vol.

Dans un mode de réalisation, les informations physiologiques comprennent un ou plusieurs des paramètres comprenant (ordre indifférent) : le suivi du regard comprenant le suivi du mouvements des yeux et/ou les fixations du regard (en anglais « Nearest Neighbor index » ou NRI), le taux de cortisol récupéré au niveau de la salive par exemple (« Hypothalamus Pituitary Adreanal » ou HPA en anglais), le rythme cardiaque, la variabilité de ce rythme cardiaque (« Heart Rate Variability » en anglais, acronyme HRV), un ou plusieurs marqueurs de l'activité du système parasympathique, le rythme respiratoire, la température de la peau, le niveau de sudation, la conductivité de la peau (en anglais « galvanic skin response » ou GSR), la dilatation pupillaire (en anglais « pupilometry » ou « Index of Cognitive Activity (ICA) »), un signal ECG (électrocardiographie), un signal EEG (électroencéphalographie), un signal MEG (magnétoencéphalographie), un signal fNIR (en anglais « functional near-infrared imaging ») ou un signal fMRI (en anglais « functional magnetic resonance »).

La détermination de l'état physiologique du pilote peut comprendre des mesures directes et/ou indirectes. Les mesures directes peuvent notamment comprendre une ou plusieurs mesures directes du rythme cardiaque et/ou ECG (électrocardiogramme) et/ou EEG (électroencéphalogramme) et/ou de la transpiration et/ou du rythme de la respiration du pilote. Les mesures indirectes peuvent notamment comprendre des estimations de l'excitation ou de la fatigue ou du stress du pilote, lesquels états peuvent en particulier être corrélées aux phases de vol ou à d'autres paramètres.

Dans un mode de réalisation de l'invention, la montre connectée comprend un ou plusieurs capteurs biométriques, configuré pour l'authentification du pilote auprès des systèmes selon l'invention (e.g. empreinte digitale, scan de l'iris, identification de la parole, empreinte de la main, reconnaissance faciale, etc).

Dans un mode de réalisation, des capteurs biométriques et des capteurs physiologiques peuvent être utilisés en combinaison.

### Commande à haut niveau d'abstraction

Dans un mode de réalisation de l'invention, l'invention permet d'effectuer des requêtes vers les différents sous-systèmes par l'utilisation de paramètres ou de commandes de vol à haut niveau d'abstraction. Par exemple, une action selon l'état de l'art peut impliquer de renseigner une même donnée opérationnelle dans deux sous systèmes distincts. Selon un mode de réalisation de l'invention, il peut être utilisé un paramètre de vol (e.g. valeur de vitesse) ou une commande de vol intégrée unique (e.g. déroutement). En effet, selon un mode de réalisation de l'invention la donnée requise peut être dupliquée vers les deux sous systèmes, diminuant le risque que l'utilisateur fasse une erreur en renseignant lui-même les deux sous système considérés.

Au contraire de la fusion d'informations (qui vise notamment à supprimer des informations peu utiles), une commande de vol intégrée depuis un client léger vise une abstraction de haut niveau, i.e. une pluralité de paramètres de vol à laquelle va correspondre une pluralité de requêtes élémentaires vers plusieurs équipements avioniques.

Dans un mode de réalisation, une commande de vol ou un paramètre de vol peut comprendre ou correspondre à un ou plusieurs appels à des API (*Application Programming Interface*). La granularité des services de cette API peut en particulier être cohérente avec les paramètres que l'utilisateur peut avoir besoin d'émettre afin de réaliser une mission opérationnelle. Plus précisément, pour un besoin opérationnel donné, il peut être besoin d'agir avec plusieurs fonctions ou sous-systèmes, parfois pour effectuer une même action sur plusieurs fonctions ou sous-systèmes, avec le risque d'erreurs humaines que cela induit.

Dans un mode de réalisation, le procédé comprend une étape consistant à déterminer les requêtes élémentaires associées avec un paramètre de vol reçu. Par suite, les différentes requêtes élémentaires sont communiquées vers les différents équipements avioniques concernés (éventuellement en les dupliquant). Dans un mode de réalisation, un retour fonctionnel unique est fourni, résultant lui aussi de la combinaison des retours ou réponses individuelles aux requêtes élémentaires. Le retour fonctionnel unique peut être porteur de sens opérationnel pour l'utilisateur. Ce mode de réalisation permet de diminuer les erreurs humaines.

Dans les modes de réalisation dans lesquels ces paramètres de vol sont implémentés via l'application centrale, cette dernière peut éviter aux applications locales de se charger des requêtes élémentaires, ce qui peut contribuer à simplifier les applications locales (mutualisation de besoins).

### Exemple d'envoi de paramètre de vol par oreillette

Un mode de réalisation dans lequel un ou plusieurs paramètres de vol sont communiqués par une oreillette connectée est décrit ci-après. Une oreillette connectée (e.g. Bluetooth BLE, ou Bluetooth version 5, de portée 4 fois supérieure à Bluetooth V4.2, oreillette Wifi ou autre, etc) ou connectée à courte distance via un téléphone en Wifi qui se connecte en wifi à l'application centrale. Dans ce mode de réalisation, des messages audio peuvent être diffusés depuis l'application centrale vers les oreillettes connectées. Dans un mode de réalisation, une oreillette est équipée d'un microphone et le procédé peut comprendre une étape consistant à traiter les commandes vocales (e.g. identification vocale, reconnaissance vocale, etc), par l'application centrale. Les messages audio peuvent servir de support à la communication d'informations générales (par exemple le passage en croisière), à des notifications impliquant une action en retour de la part du membre de l'équipage destinataire du message (par exemple un changement planifié de niveau de vol imminent), ou à des messages d'alertes (par exemple la traversée prochaine d'une zone de turbulences). Ces messages peuvent prendre la forme de sons standardisés ou de messages parlés (voix humaine). Les messages parlés peuvent, soit provenir des utilisateurs connectés qui communiquent entre eux à travers leurs micros et via l'application centrale, soit résulter d'une synthèse vocale gérée par l'application centrale, afin d'exprimer humainement et oralement une information en provenance de l'avionique (par exemple la mise à jour de l'heure d'atterrissage prévue).

Dans un mode de réalisation, des commandes vocales peuvent être manipulées. Ces commandes peuvent concerner des commandes d'administration des interactions entre l'application centrale et l'oreillette, par exemple l'activation ou la désactivation des notifications. Dans certains modes de réalisation des commandes vocales peuvent être utilisées (e.g. reconnaissance vocale pour transformer les ordres parlés des utilisateurs en commandes de vol ou paramètres de vol prévus dans les API de l'application centrale, qui les transfère ensuite aux systèmes avioniques concernés.

*Légère, l'implémentation par oreillettes est avantageuse car très peu* intrusive pour les mouvements de l'utilisateur, qui permet de rester connecté à l'avionique et au reste de l'équipage tout le temps et depuis n'importe où dans l'avion. Tous les personnels (des pilotes aux PNC) sont des utilisateurs possibles des oreillettes à un moment ou à un autre de leur mission.

### Autres systèmes d'envoi de paramètre de vol

Dans un mode de réalisation, la communication de paramètres de vol se fait avec une paire de lunettes connectées. Le port de lunettes connectées (ou de lentilles, même à faible résolution) présente plusieurs avantages : un affichage personnel (pas nécessairement limité ou exigu puisque des écrans virtuels de grandes dimensions peuvent être affichées), une assurance d'authentification puisque les iris du porteur peuvent être testés, des voies audio (entrée/sortie), une intrusivité limitée, etc. Une caméra portée permet en outre une capture de la vue subjective de son porteur, des mesures de densité visuelle, la reconnaissance faciale de passagers, des options de réalité augmentée, etc.

Dans un mode de réalisation, la communication de paramètres de vol se fait avec une montre connectée. Le port d'une montre connectée confère certains avantages: une certaine assurance d'authentification (port physique par l'utilisateur, code de verrouillage, mesures biométriques de signature, etc), un affichage personnel, des voies audio et/ou vidéo (entrée/sortie), une intrusivité limitée, etc. Une caméra ou un projecteur embarqué permettent en outre une certaine capture de la vue subjective de son porteur, la reconnaissance faciale de passagers, des options de réalité augmentée, etc.

Dans certains modes de réalisation, plusieurs types de clients légers sont déployés, i.e. une combinaison d'oreilles, de téléphones portables, de lunettes connectées, de montres connectées, etc. Avantageusement, les interactions sont multimodales.

### Interfaces homme-machine

L'interface homme-machine selon l'invention peut également comprendre des interfaces ou périphériques d'entrée. Dans un développement, le dispositif comprend des moyens de sélection d'une ou plusieurs portions de l'affichage virtuel. Les pointages des interfaces homme-machine (IHM) ou de portions de ces interfaces ou informations peuvent être accessibles via différents dispositifs, par exemple un dispositif de pointage de type « souris » ou une désignation basée sur un pointage manuel ; via des interfaces d'acquisition (bouton, molette, joystick, clavier, télécommande, capteurs de mouvement, microphone, etc.), via des interfaces combinées (écran tactile, commande à retour d'effort, gants ou *glove,* etc.). Les interfaces homme-machine d'entrée ou de sélection peuvent en effet comprendre une ou plusieurs interfaces de sélection (menus, pointeurs, etc.), interfaces graphiques, interfaces vocales, interface gestuelle et de position. Dans un mode de réalisation avantageux, une sélection peut être effectuée par le regard (e.g. durée de fixation en excès d'un seuil de durée prédéfini, clignement de l'oeil, commande vocale concomitante, contraction d'un muscle, commande au pied, etc.). Dans un mode de réalisation, une sélection peut être effectuée par un ou plusieurs mouvements de tête.

L'affichage sélectionné peut être divers (nature) et une pluralité d'espaces ou de surfaces (e.g. planes, courbes, etc.) peut être mobilisée. Un affichage peut être un écran en tête basse, un HUD, une visière du casque ou un pare-brise. Un affichage peut aussi résulter d'une projection. Dans certains modes de réalisation, les espaces de projection sont sélectionnés de manière « opportuniste » (par exemple, les espaces non utilisés du tableau de bord sont utilisés e.g. les montants ou les espaces interstitiels entre les écrans). Dans un mode de réalisation, un ou plusieurs espaces peuvent être prédéfinis pour des projections (ils peuvent être intentionnellement dédiés à cette tâche). Par exemple, une zone libre du cockpit peut permettre à un projecteur d'afficher de l'information. Généralement, rien ne restreint cette liberté de projection qui peut être effectuée sur n'importe quel type de support (matériel e.g. plastiques, tissus, verre, etc. y compris corps humain), étant donné que les systèmes de projection peuvent adapter leurs affichage de manière à se conformer à l'environnement et à produire des images stables et formées, connaissant le point de vue subjectif cible.

Il est décrit un produit programme d'ordinateur, ledit programme d'ordinateur comprenant des instructions de code permettant d'effectuer une ou plusieurs des étapes du procédé, lorsque ledit programme est exécuté sur un ordinateur.

A titre d'exemple d'architecture matérielle adaptée à mettre en oeuvre l'invention, un dispositif peut comporter un bus de communication auquel sont reliés une unité centrale de traitement ou microprocesseur (CPU, acronyme de « Central Processing Unit » en anglais), lequel processeur peut être "multi-core" ou "many-core"; une mémoire morte (ROM, acronyme de « Read Only Memory » en anglais) pouvant comporter les programmes nécessaires à la mise en oeuvre de l'invention; une mémoire vive ou mémoire cache (RAM, acronyme de « Random Access Memory » en anglais) comportant des registres adaptés à enregistrer des variables et paramètres créés et modifiés au cours de l'exécution des programmes précités ; et une interface de communication ou E/S (I/O acronyme de « Input/ouput » en anglais) adaptée à transmettre et à recevoir des données. Dans le cas où l'invention est implantée sur une machine de calcul reprogrammable (par exemple un circuit FPGA), le programme correspondant (c'est-à-dire la séquence d'instructions) peut être stocké dans ou sur un médium de stockage amovible (par exemple une carte SD, ou un stockage de masse tel que un disque dur e.g. un SSD) ou non-amovible, volatile ou non-volatile, ce médium de stockage étant lisible partiellement ou totalement par un ordinateur ou un processeur. La référence à un programme d'ordinateur qui, lorsqu'il est exécuté, effectue l'une quelconque des fonctions décrites précédemment, ne se limite pas à un programme d'application s'exécutant sur un ordinateur hôte unique. Au contraire, les termes programme d'ordinateur et logiciel sont utilisés ici dans un sens général pour faire référence à tout type de code informatique (par exemple, un logiciel d'application, un micro logiciel, un microcode, ou toute autre forme d'instruction d'ordinateur, comme des web services ou SOA ou via des interfaces de programmation API) qui peut être utilisé pour programmer un ou plusieurs processeurs pour mettre en oeuvre des aspects des techniques décrites ici. Les moyens ou ressources informatiques peuvent notamment être distribués ("Cloud computing"), éventuellement avec ou selon des technologies de pair-à-pair et/ou de virtualisation. Le code logiciel peut être exécuté sur n'importe quel processeur approprié (par exemple, un microprocesseur) ou coeur de processeur ou un ensemble de processeurs, qu'ils soient prévus dans un dispositif de calcul unique ou répartis entre plusieurs dispositifs de calcul (par exemple tels qu'éventuellement accessibles dans l'environnement du dispositif). Des technologies de sécurisation (crypto-processeurs, authentification éventuellement biométrique, chiffrement, carte à puce, etc) peuvent être utilisées.

## Revendications

1. Procédé pour la communication de données de type avionique à un dispositif de type non-avionique situé en dehors du cockpit d'un aéronef, le procédé comprenant les étapes consistant à :
- recevoir une requête visant à recevoir des données de type avionique depuis un ou plusieurs systèmes avioniques situés à un point de référence dans l'aéronef de la part d'un dispositif client non-avionique;
- déterminer un paramètre de liaison entre le dispositif client non-avionique et le point de référence;
- ajuster l'envoi de données en réponse à ladite requête en fonction du paramètre de liaison déterminé.

2. Procédé selon la revendication 1, le paramètre de liaison comprenant un ou plusieurs paramètres d'espace et/ou de temps caractérisant la relation entre le dispositif client non-avionique et un ou plusieurs systèmes avioniques, le paramètre de liaison comprenant notamment la distance physique entre le dispositif client non-avionique et le point de référence dans l'aéronef, et/ou la qualité de service associée aux communications sans-fil entre le dispositif client non-avionique et le point de référence dans l'aéronef, et/ou d'un droit d'accès prédéfini associé à un dispositif avionique et/ou au dispositif client non-avionique.

3. Procédé selon l'une quelconque des revendications précédentes, le point de référence étant le cockpit de l'aéronef ou d'un autre aéronef, ou une porte de secours, ou un local de repos, ou un siège passager.

4. Procédé selon l'une quelconque des revendications précédentes, la requête étant reçue par un point d'accès sans-fil associé à une passerelle sécurisée, ladite passerelle sécurisée déterminant les droits d'accès aux données avioniques par le dispositif client non-avionique.

5. Procédé selon la revendication 4, la passerelle sécurisée pour l'échange de données entre système avionique et système non-avionique implémentant des fonctionnalités comprenant des règles de routage, une gestion des ports de communication pour autoriser ou interdire des communications et une gestion de couches protocolaires.

6. Procédé selon la revendication 1, l'étape consistant à ajuster l'envoi de données étant en outre fonction des capacités d'affichage et/ou de calcul du dispositif client non-avionique.

7. Procédé selon l'une quelconque des revendications précédentes, l'envoi de données étant réduit ou stoppé ou augmenté ou modifié si la distance déterminée est au delà ou en deçà de seuils ou de plages de seuils prédéfinis.

8. Procédé selon la revendication 2, l'étape consistant à déterminer la distance ou la position entre le dispositif non-avionique et le cockpit de l'aéronef étant effectuée par application d'une ou de plusieurs technologies comprenant le positionnement par empreinte radio FM, par utilisation d'un réseau de balises Bluetooth BLE, par l'utilisation d'un réseaux de balise RFID, par l'utilisation d'un tapis de sol avec des réseaux de capteurs embarqués, par combinaison de technologies RFID et WLAN, par reconnaissance d'images, par ultrasons et techniques d'angle d'arrivée, par utilisation d'une centrale à inertie, et/ou de positionnement par mesure de champ magnétique ambiant.

9. Procédé selon la revendication 1, les communications étant unidirectionnelles depuis des systèmes avioniques vers le dispositif non-avionique, lesdites communications comprenant une ou plusieurs notifications selon une ou plusieurs modalités, comprenant une modalité visuelle, sonore et/ou vibratile.

10. Procédé selon la revendication 1, les communications étant bidirectionnelles entre les systèmes avioniques et le dispositif non-avionique.

11. Procédé selon l'une quelconque des revendications précédentes, dans lequel le dispositif non-avionique comprend une oreillette et/ou une montre connectée et/ou une paire de lunettes connectées.

12. Procédé selon l'une quelconque des revendications précédentes, comprenant en outre une étape consistant à envoyer des données depuis le dispositif client non-avionique vers les systèmes avioniques du cockpit, l'envoi étant sans-fil depuis des zones d'espace prédéfinies et non publiques dans l'aéronef, et/ou par l'intermédiaire de liaisons filaires non accessibles aux passagers.

13. Procédé selon l'une quelconque des revendications précédentes, comprenant en outre une ou plusieurs des étapes consistant à conditionner l'envoi de données à l'enregistrement préalable du dispositif non-avionique auprès du cockpit, à procéder à une étape d'authentification biométrique, à vérifier des conditions physiologiques du porteur du dispositif non-avionique, et/ou à chiffrer les communications.

14. Procédé selon l'une quelconque des revendications précédentes, dans lequel un système avionique est associé avec un taux de panne physique inférieur et une vérification logique supérieure à ceux d'un système de type non-avionique.

15. Procédé selon l'une quelconque des revendications précédentes, dans lequel un système avionique est associé avec une exhaustivité des tests et/ou de vérifications supérieure à ceux d'un système de type non-avionique.

16. Produit programme d'ordinateur, ledit programme d'ordinateur comprenant des instructions de code permettant d'effectuer les étapes du procédé selon l'une quelconque des revendications 1 à 15, lorsque ledit programme est exécuté sur un ordinateur.

17. Système pour la mise en oeuvre du procédé selon les revendications 1 à 15 pour la communication de données de type avionique à un dispositif de type non-avionique situé en dehors du cockpit d'un aéronef.
